# EUROPEAN PATENT SPECIFICATION

(11) **EP 1 977 238 B1**
(45) Date of publication and mention of the grant of the patent: **28.09.2016**
(21) Application number: 06850597.3
(22) Date of filing: 21.12.2006
(51) Int. Cl.: G01N 33/68, G01N 33/574

(54) **SOLUBLE HUMAN M-CSF RECEPTOR AND USES THEREOF**
LÖSLICHER MENSCHLICHER M-CSF-REZEPTOR UND VERWENDUNGEN DAVON
RÉCEPTEUR SOLUBLE HUMAIN DU FACTEUR M-CSF ET UTILISATIONS DE CELUI-CI

(30) Priority: 22.12.2005 US 753218 P
(43) Date of publication of application: 08.10.2008
(73) Proprietor: Novartis AG, 4056 Basel (CH); XOMA Technology Ltd., Berkeley, CA 94710 (US)
(72) Inventor: LIU, Cheng, Richmond, CA 94803 (US); DEUTER-REINHARD, Maja, Alameda, CA 94501 (US); KUNICH, John, Emeryville, CA 94662-8097 (US); KAVANAUGH, Michael, Emeryville, CA 94662-8097 (US)
(74) Representative: Marshall, Cameron John
(86) International application number: PCT/US2006/048879
(87) International publication number: WO 2007/120252

(56) References cited:
- WO-A-89/03687
- WO-A-2004/045532
- WO-A-2004/075775
- RAO Q ET AL: "Determination of serum soluble macrophage colony-stimulating factor receptor levels in patients with hematological diseases" CHINESE JOURNAL OF CANCER RESEARCH 2001 CHINA, vol. 13, no. 3, 2001, pages 185-189, XP009094774 ISSN: 1000-9604
- STORGA D ET AL: "C-FMS IS PRESENT IN PRIMARY TUMOURS AS WELL AS IN THEIR METASTASES IN BONE MARROW" INTERNATIONAL JOURNAL OF EXPERIMENTAL PATHOLOGY, vol. 73, no. 4, 1992, pages 527-533, XP009094714 ISSN: 0959-9673
- BAIOCCHI G ET AL: "EXPRESSION OF THE MACROPHAGE COLONY-STIMULATING FACTOR AND ITS RECEPTOR IN GYNECOLOGIC MALIGNANCIES" CANCER, vol. 67, no. 4, 1991, pages 990-996, XP002466462 ISSN: 0008-543X
- KAUMA S W ET AL: "COLONY-STIMULATING FACTOR-1 AND C-FMS EXPRESSION IN HUMAN ENDOMETRIAL TISSUES AND PLACENTA DURING THE MENSTRUAL CYCLE AND EARLY PREGNANCY" JOURNAL OF CLINICAL ENDOCRINOLOGY AND METABOLISM, ENDOCRINE SOCIETY, CHEVY CHASE, MD, vol. 73, no. 4, 1 January 1991 (1991-01-01), pages 746-751, XP009103111 ISSN: 0021-972X
- FUJIMOTO J ET AL: "Induction of M-CSF receptor and its mRNA, and activation of tyrosine kinase in peripheral monocytes by oestradiol-17 beta and progesterone" ANNALS OF CLINICAL BIOCHEMISTRY, BRITISH MEDICAL ASSOCIATION, LONDON, GB, vol. 32, no. Part 4, 1 July 1995 (1995-07-01), pages 399-404, XP009103109 ISSN: 0004-5632
- SHINETUGS B ET AL: "Colony stimulating factor-1 concentrations in blood and follicular fluid during the human menstrual cycle and ovarian stimulation: possible role in the ovulatory process." HUMAN REPRODUCTION (OXFORD, ENGLAND) MAY 1999, vol. 14, no. 5, May 1999 (1999-05), pages 1302-1306, XP002488257 ISSN: 0268-1161
- COHEN P E ET AL: "Macrophages: important accessory cells for reproductive function." JOURNAL OF LEUKOCYTE BIOLOGY NOV 1999, vol. 66, no. 5, November 1999 (1999-11), pages 765-772, XP002488258 ISSN: 0741-5400
- PAMPFER S ET AL: "Expression of colony-stimulating factor-1 (CSF-1) messenger RNA in human endometrial glands during the menstrual cycle: molecular cloning of a novel transcript that predicts a cell surface form of CSF-1" MOLECULAR ENDOCRINOLOGY, BALTIMORE, MD, US, vol. 5, no. 12, 1 December 1991 (1991-12-01), pages 1931-1938, XP009103112 ISSN: 0888-8809

## Description

### TECHNICAL FIELD

This invention relates to methods of diagnosing breast cancer by detecting in patient samples a naturally occurring soluble fragment of the receptor for human M-CSF which is capable of binding M-CSF.

### BACKGROUND OF THE INVENTION

An estimated 1.4 million new cases of cancer occur every year, and an estimated 0.6 million people die from cancer every year. With improvements in detection and treatment, many of these patients survive for significant periods of time. As of January 1, 2002, there were an estimated 10.1 million cancer survivors, representing approximately 3.4% of the population. Of these cancer survivors, breast (22%), prostate (18%), colorectal (10%) and gynecologic (10%) are the most common cancer sites.

In general, cancer morbidity and mortality increases significantly if it is not detected early in its progression. Treatment decisions are often linked to the stage of cancer when diagnosed. Thus, there is a great need for sensitive and accurate methods for diagnosing, staging, predicting prognosis, and monitoring progress or regression of cancer during therapy.

Macrophage colony-stimulating factor (M-CSF or CSF-1) regulates the survival, proliferation, and differentiation of macrophages and their precursors and osteoclasts and their precursors. M-CSF activity is tightly controlled through mechanisms regulating gene expression of M-CSF and its membrane-bound receptor (M-CSFR), as well as by receptor-mediated endocytosis, metabolic processing, and inhibition of downstream signaling.

M-CSF is expressed in stromal cells, osteoblasts, and other cells. It is also expressed in breast, uterine, and ovarian tumor cells. The extent of expression in these tumors correlates with high grade and poor prognosis (Kacinski Ann. Med. 27: 79-85 (1995); Smith et al., Clin. Cancer Res. 1: 313-25 (1995)). In breast carcinomas, M-CSF expression is prevalent in invasive tumor cells as opposed to the intraductal (pre-invasive) cancer (Scholl et al., J. Natl. Cancer Inst. 86: 120-6 (1994)). In addition, M-CSF is shown to promote progression of mammary tumors to malignancy (Lin et al., J. Exp. Med. 93: 727-39 (2001)).

Human membrane-bound M-CSF receptor is encoded by the *c-fms* proto-oncogene and is a protein tyrosine-kinase transmembrane receptor, a Type I membrane protein, expressed in bone marrow and in differentiated blood mononuclear cells. The M-CSF receptor belongs to the Tyr protein kinase family and the CSF-1/PDGF receptor subfamily. It contains five Ig-like C2-type (immunoglobulin-like) domains, one transmembrane domain, and an intracellular interrupted Src-related domain with two kinase domains. M-CSF typically binds to its receptor in order to exert a biological effect. M-CSF binding to c-fmsleads to homo-dimerization of the receptor, which activates the cytoplasmic kinase domain, causing autophosphorylation and phosphorylation of other cellular proteins. These events initiate a cascade of signal transduction pathways including the JAK/STAT, P13K, and ERK pathways that result in a variety of cellular responses: mitosis, secretion of cytokines, membrane ruffling, and regulation of transcription of M-CSFR. (Hamilton J. A., J Leukoc Biol.,62(2):145-55 (1997); Hamilton J, A., Immuno Today., 18(7): 313-7(1997); Fixe and Praloran, Cytokine 10: 32-37 (1998)).

### SUMMARY OF THE INVENTION

The invention provides an *in vitro* method of diagnosing breast cancer comprising the step of:
(a) analyzing a fluid sample from a patient for level of soluble M-CSF receptor, wherein a level of soluble M-CSF receptor above a threshold is correlated with the presence of breast cancer and a level below said threshold indicates that the patient is unlikely to have cancer.

The invention also provides an *in vitro* method of determining prognosis in a subject afflicted with breast cancer comprising the step of:
(a) analyzing a fluid sample from a patient for level of soluble M-CSF receptor, wherein a level of soluble M-CSF receptor above a threshold indicates that the patient is likely to have a poor prognosis and a level below said threshold indicates that the patient is likely to have a good prognosis.

The invention also provides an *in vitro* method of monitoring breast cancer therapy in a subject afflicted with breast cancer comprising the steps of:
(a) analyzing a fluid sample from a patient for level of soluble M-CSF receptor prior to the initiation of treatment with a cancer therapeutic; and
(b) analyzing said fluid sample after the initiation of the treatment with the cancer therapeutic,
wherein a reduction in the level of soluble M-CSF receptor after the initiation of the treatment with the cancer therapeutic indicates the patient is receiving a therapeutically effective dose of the cancer therapeutic.

The invention also provides use of a kit in a method of the invention, the kit comprising:
(a) a first antibody that specifically binds in a fluid sample shM-CSFR; and
(b) an M-CSFR standard containing a known quantity of M-CSFR.

### SUMMARY OF THE DISCLOSURE

The materials and methods disclosed herein fulfill the aforementioned and other related needs in the art. The discovery of naturally occurring soluble fragment(s) of human M-CSF (CSF-1) receptor (lacking a transmembrane domain) makes possible the recombinant production of such naturally occurring fragments for therapeutic uses where neutralization of M-CSF activity is desired.

Disclosed herein is a fusion protein comprising the aforementioned polypeptide and a second polypeptide, wherein the aforementioned polypeptide and the second polypeptide are linked such that the normal biological activity of the aforementioned polypeptide is not compromised. Exemplary fusion proteins include immunoglobulin Fc fusions.

Also disclosed herein is a pharmaceutical composition comprising a specific naturally occurring form of soluble M-CSF receptor or fusion protein, as well as methods of using such pharmaceutical compositions to treat cancer or metabolic bone diseases associated with increased osteoclast activity.

Also disclosed herein is a method of diagnosing cancer comprising the step of: (a) analyzing a fluid sample from a patient for level of soluble M-CSF receptor, wherein a level of soluble M-CSF receptor above a threshold is correlated with the presence of cancer and a level below said threshold indicates that the patient is unlikely to have cancer. Also disclosed herein is a method of determining prognosis in a subject afflicted with cancer comprising the step of: (a) analyzing a fluid sample from a patient for level of soluble M-CSF receptor, wherein a level of soluble M-CSF receptor above a threshold indicates that the patient is likely to have a poor prognosis and a level below said threshold indicates that the patient likely has a good prognosis. Also disclosed herein is a method of monitoring cancer therapy in a subject afflicted with cancer comprising the steps of (a) analyzing a fluid sample from a patient for level of soluble M-CSF receptor prior to the initiation of treatment with a cancer therapeutic; and (b) analyzing the fluid sample after the initiation of the treatment with the cancer therapeutic, wherein a reduction in the level of soluble M-CSF receptor after the initiation of the treatment with the cancer therapeutic indicates the patient is receiving a therapeutically effective dose of the cancer therapeutic.

It is contemplated that any of the preceding methods involving diagnosis, prognosis or monitoring of cancer therapy can be carried out for any cancer. Exemplary cancers include breast, lung, renal, multiple myeloma, thyroid, prostate, adenocarcinoma, blood cell malignancies, including leukemia and lymphoma; head and neck cancers; gastrointestinal cancers, including esophageal cancer, stomach cancer, colon cancer, intestinal cancer, colorectal cancer, rectal cancer, pancreatic cancer, liver cancer, cancer of the bile duct or gall bladder; malignancies of the female genital tract, including ovarian carcinoma, uterine endometrial cancers, vaginal cancer, and cervical cancer; bladder cancer; brain cancer, including neuroblastoma; sarcoma, osteosarcoma; and skin cancer, including malignant melanoma or squamous cell cancer.

Also disclosed herein is a method of monitoring menstrual cycle in a female comprising the step of: (a) analyzing a fluid sample from a female patient for level of soluble M-CSF receptor, wherein a level of soluble M-CSF receptor above a threshold indicates that the patient is likely fertile and a level below the threshold indicates that the patient is likely not fertile. Also disclosed herein is a method of detecting endometrial proliferation in a female comprising similar steps, wherein a level of soluble M-CSF receptor above a threshold is correlated with abnormal endometrial proliferation, e.g. endometriosis, and a level below the threshold indicates that the patient likely has a normal endometrial proliferative state.

Exemplary fluid samples that may be analyzed according to any of the preceding methods regarding cancer or endometrial proliferation include urine, plasma, or serum.

Also disclosed is a processor, a computer readable memory, and a routine stored on the computer readable memory and adapted to be executed on the processor to perform any of the preceding methods, and/or to generate as output the detected level of soluble M-CSF receptor and a threshold or range of threshold levels considered "normal", such that levels outside the "normal" range correlate with one or more of the conditions described above. Also disclosed is computer readable media containing programs or routines to perform similar functions. Examples of suitable computing systems, environments, and/or configurations include personal computers, server computers, hand-held or laptop devices, multiprocessor systems, microprocessor-based systems, set top boxes, programmable consumer electronics, network PCs, minicomputers, mainframe computers, distributed computing environments that include any of the above systems or devices, or any other systems known in the art.

Also disclosed is a kit comprising: (a) a first antibody that specifically binds to soluble M-CSF receptor; and (b) an M-CSF receptor standard containing a known quantity of M-CSF receptor. The aforementioned kit is contemplated wherein the first antibody is linked to a detectable label. The label may be an enzyme. The kit may further comprise a substrate from which the enzyme releases a detectable signal. The aforementioned kit may further comprise a second antibody that binds to soluble M-CSF receptor. The aforementioned kit may further comprise a second antibody that binds to the first antibody. The aforementioned kit may further comprise a second antibody that binds to soluble M-CSF receptor.

The soluble M-CSF receptor levels may detect the presence of, predict prognosis or severity of, or monitor the progression of a disease selected from the group consisting of osteolytic diseases associated with relatively increased osteoclast activity or the osteoclastic/osteoblastic process required for bone remodeling, including metabolic bone diseases, endocrinopathies (hypercortisolism, hypogonadism, primary or secondary hyperparathyroidism, hyperthyroidism), hypercalcemia, deficiency states (rickets/osteomalacia, scurvy, malnutrition), chronic diseases (malabsorption syndromes, chronic renal failure (renal osteodystrophy), chronic liver disease (hepatic osteodystrophy)), drugs (glucocorticoids (glucocorticoid-induced osteoporosis), heparin, alcohol), and hereditary diseases (osteogenesis imperfecta, homocystinuria), cancer, osteoporosis, periprosthetic bone loss (for example, due to a foreign implant in bone or joint replacement with a prosthesis, including total hip arthroplasty), inflammation of bone associated with arthritis and rheumatoid arthritis, periodontal disease, fibrous dysplasia, and/or Paget's disease.

### BRIEF DESCRIPTION OF THE DRAWINGS

Figure 1 shows the standard curve used to quantify the concentration of soluble human M-CSF receptor.
Figure 2 shows the detection of soluble human M-CSF receptor in human serum samples.
Figure 3 shows the binding of human M-CSF by soluble c-fins in human serum.
Figure 4 shows the affinity analysis of soluble human M-CSF receptor.
Figure 5 shows the immunoprecipitation (IP) of soluble human M-CSF receptor.
Figure 6 shows the deglycosylation of soluble human M-CSF receptor.
Figure 7 shows the SDS-PAGE analysis of soluble human M-CSF receptor.
Figure 8 shows the sequence analysis of a peptide (SEQ ID NO: 1) from Trypsin digestion of deglycosylated soluble human M-CSF receptor.
Figure 9 shows the detection of soluble human M-CSF receptor in human urine samples.
Figure 10 shows the soluble human M-CSF receptor level in urine samples during menstrual cycle.
Figure 11 shows the level of soluble human M-CSF receptor in breast cancer patient samples.
Figure 12 shows the correlation between soluble human M-CSF receptor and M-CSF level in breast cancer patient serum samples.
Figure 13 shows that soluble M-CSF receptor was found in Cynomulgus and rhesus monkeys.
Figure 14 shows the expression of soluble human M-CSF receptor during osteoclast differentiation depends on M-CSF activity.
Figure 15 shows that soluble human M-CSF receptor expression follows TRAP activity of osteoclast.
Figure 16 shows that removal of M-CSF from culture media stimulates soluble human M-CSF receptor expression of differentiated osteoclasts.
Figure 17 shows the effect of Zometa and M-CSF neutralizing antibody on the expression of soluble human M-CSF receptor in differentiated osteoclasts.

### DETAILED DESCRIPTION

The present invention is based on the discovery of a naturally occurring soluble form of human M-CSF receptor, which is normally a membrane-bound receptor. Concentrations of this soluble form of the receptor (shM-CSFR) have been shown to be correlated with cancer, osteoclast differentiation and menstrual cycle. Increasing concentrations of shM-CSFR have been shown to be correlated with severity of cancer and cellular proliferative states.

Thus, disclosed herein are methods of detecting or monitoring cellular proliferative states, particularly proliferative states found in cancer, endometriosis, and the buildup of uterine lining during a female's fertile period. The methods include methods of diagnosing, staging, predicting prognosis or severity, and monitoring progress or regression of such proliferative states, e.g. during and/or after cancer chemotherapy, endometriosis therapy or during the menstrual cycle.

Also disclosed are methods of detecting or monitoring osteoclast proliferative states, particularly those involving osteoclast differentiation. The methods include methods of diagnosing, staging, predicting prognosis or severity, and monitoring progress or regression of metabolic bone diseases during and/or after therapy.

Also disclosed is a purified shM-CSFR. The shM-CSFR is purified from human serum or urine and sequenced according to methods known in the art. For example, affinity chromatography with a specific antibody against c-fins is used for affinity purification. The antibodies (Duo Set Elisa development system hM-CSF R (R&D systems, Cat# DY329)) used in the soluble receptor ELISA assay described herein (see, e.g., Example 1) are used. Alternatively or additionally, since the shM-CSFR has been found to be glycosylated, a lectin affinity chromatography step is used which purifies glycosylated proteins. An ion exchange chromatography can also be used to further purify the shM-CSFR. Before subjecting the purified shM-CSFR to protein sequencing, a de-glycosylation reaction is performed using methods known in the art and described herein (see, e.g., Example 1). A combined method of N-terminal sequencing, peptide mapping, and mass-spectroscopy is used to determine the full sequence of the shM-CSFR.

Any methods of detecting a soluble protein in a patient sample may be used to detect the presence or relative quantity of shM-CSFR associated with any of the above-described diseases or conditions. Particularly preferred methods include any antibody-based immunoassays known in the art, such as ELISA (Enzyme-linked immunosorbent assay), RIA. (Radioimmunoassay), LIA (luminescent immunoassay), and FIA (fluorescent immunoassay). The antibodies - either primary or secondary -may be labelled with any labels known in the art, including radioisotopes (e.g., ¹²⁵I), fluorescent dyes (e.g., FITC) or enzymes (e.g., HRP or AP) which catalyse fluorogenic or luminogenic reactions.

Steps of such methods include analyzing a fluid sample from a patient for the presence of or relative level of shM-CSF receptor, wherein a level of soluble M-CSF receptor above a threshold indicates that the patient is likely to have a disease state or condition and a level below said threshold indicates that the patient is unlikely to have such disease state or condition. Sensitivity and specificity can each be set at any desired level, e.g. 855, 90%, 95%, 96% 97%, 98%, 99% or higher.

Any patient samples may be used, but preferred are fluid samples such as blood (including whole blood, plasma or serum), urine, saliva, cerebrospinal fluid, ascites, pleural fluid, aspirates, or any other bodily effusion or secretion.

As used herein, the term "diagnosis" means detecting a disease or disorder or determining the stage or degree of a disease or disorder. The term "diagnosis" also encompasses detecting a predisposition to a disease or disorder, determining the therapeutic effect of a drug therapy, or predicting the pattern of response to a drug therapy. The diagnosis methods of the present invention may be used independently, or in combination with other diagnosing and/or staging methods known in the medical art for a particular disease or disorder. Thus, the method of diagnosis may be conducted by detecting, in a patient, the concentrations of shM-CSFR disclosed herein using any one of the methods described herein, and determining whether the patient has an aberrant concentration of the protein. The diagnostic methods may be applied to a subset of patients suspected of having the disease or condition because of other clinical signs or symptoms of disease.

"Prognosis" is a forecast as to the probable outcome of an attack or disease, and/or the prospect as to recovery from a disease as indicated by the nature and symptoms of the disease. By way of example, a poor prognosis forecasts an unlikely recovery and possibly death, while a good prognosis forecasts a likely recovery and possibly complete return of health.

The term "threshold" means a quantitative or qualitative measure (e.g., concentration, level, activity, etc.) of a known substance (e.g., a molecule, compound, etc.) under defined conditions that correlates with presence or absence of a condition. In this way, the measure of the known substance under various conditions can be compared to the threshold measure to assist in determining whether a patient is suffering from the condition.

The term "standard" refers to a set range of specific measures (e.g., concentration) of a known substance (e.g., M-CSFR) under defined conditions.

The term "analyze" means to detect or measure a component in a complex substance (e.g., a fluid such as serum). "Monitoring" is the process of periodically observing or testing the effect of a procedure (e.g., a procedure for treating a patient afflicted with cancer). "Staging" refers to prediction of the clinical stage of a cancer or other disease (e.g. early stage or advanced stage) by analyzing a marker level that has been correlated with clinical stage as determined by accepted medical practice.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma,; lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (e.g., cancer) treatment, a therapeutic agent may directly decrease the pathology of tumor cells, or render the tumor cells more susceptible to treatment by other therapeutic agents, e.g., radiation and/or chemotherapy. Treatment of patients suffering from clinical, biochemical, radiological or subjective symptoms of the disease, such as osteolysis, may include alleviating some or all of such symptoms or reducing the predisposition to the disease. The "pathology" of cancer includes all phenomena that compromise the well being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc. Thus, improvement after treatment may be manifested as decreased tumor size, decline in tumor growth rate, destruction of existing tumor cells or metastatic cells, and/or a reduction in the size or number of metastases.

"Mammal" for purposes of treatment refers to any animal classified as a mammal, including humans, domestic and farm animals, and zoo, sports, or pet animals, such as dogs, horses, cats, cows, etc. Preferably, the mammal is human.

As used herein, the phrase "metastatic cancer" is defined as cancers that have potential to spread to other areas of the body, particularly bone. A variety of cancers can metastasize to the bone, but the most common metastasizing cancers are breast, lung, renal, multiple myeloma, thyroid and prostate. By way of example, other cancers that have the potential to metastasize to bone include but are not limited to adenocarcinoma, blood cell malignancies, including leukemia and lymphoma; head and neck cancers; gastrointestinal cancers, including esophageal cancer, stomach cancer, colon cancer, intestinal cancer, colorectal cancer, rectal cancer, pancreatic cancer, liver cancer, cancer of the bile duct or gall bladder; malignancies of the female genital tract, including ovarian carcinoma, uterine endometrial cancers, vaginal cancer, and cervical cancer; bladder cancer; brain cancer, including neuroblastoma; sarcoma, osteosarcoma; and skin cancer, including malignant melanoma and squamous cell cancer. Contemplated herein is prevention and treatment of tumor-induced osteolytic lesions in bone.

As used herein, the phrase "therapeutically effective amount" refers to is meant to refer to an amount of therapeutic or prophylactic soluble fragment of the M-CSF receptor that would be appropriate for an embodiment of the present invention, that will elicit the desired therapeutic or prophylactic effect or response when administered in accordance with the desired treatment regimen.

Human "M-CSF" as used herein refers to a human polypeptide having substantially the same amino acid sequence as the mature human M-CSFα, M-CSFβ, or M-CSFγ polypeptides described in Kawasaki et al., Science 230:291 (1985), Cerretti et al., Molecular Immunology, 25:761 (1988), or Ladner et al., EMBO Journal 6:2693 (1987),. Such terminology reflects the understanding that the three mature M-CSFs have different amino acid sequences, as described above, and that the active form of M-CSF is a disulfide bonded dimer; thus, when the term "M-CSF" refers to the biologically active form, the dimeric form is intended. "M-CSF dimer" refers to two M-CSF polypeptide monomers that have dimerized and includes both homodimers (consisting of two of the same type of M-CSF monomer) and heterodimers (consisting of two different monomers). M-CSF monomers may be converted to M-CSF dimers in vitro as described in U.S. Pat. No. 4,929,700.

"Soluble human M-CSF receptor" (shM-CSFR) means a polypeptide having substantially the same amino acid sequence as the extracellular portion of M-CSFR of SEQ ID NO: 2.

### Anti-shM-CSFR antibodies

Monoclonal or polyclonal antibodies may be used in the detection methods of the present invention, although monoclonal antibodies are advantageously used because of their consistency and specificity. Rodent antibodies such as murine monoclonal antibodies are suitable for use in diagnostic methods. The modifier "monoclonal" indicates the character of the antibody as being obtained from a substantially homogeneous population of antibodies, and is not to be construed as requiring production of the antibody by any particular method.

The antigen to be used for production of antibodies may be, e.g., intact shM-CSFR or a fragment of shM-CSFR that retains the desired epitope, optionally fused to another polypeptide that allows the epitope to be displayed in its native confirmation. Alternatively, cells expressing shM-CSFR can be used to generate antibodies. Such cells can be transformed to express M-CSF or may be other naturally occurring cells that express shM-CSFR. Other forms of shM-CSFR useful for generating antibodies will be apparent to those skilled in the art.

For recombinant production of the shM-CSFR, the nucleic acid encoding it is isolated and inserted into a replicable vector for further cloning (amplification of the DNA) or for expression as is well known in the art. DNA encoding the monoclonal antibody is readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more selective marker genes, an enhancer element, a promoter, and a transcription termination sequence.

The antigen is used to generate monoclonal antibodies using any methods known in the art, e.g. the hybridoma method first described by Kohler et al., Nature, 256:495 19751, or recombinant DNA methods (see, e.g., U.S. Patent No. 4,816,567), or isolated from phage antibody libraries using the techniques described in Clackson et al., Nature, 352:624628[1991] and Marks et al., J. Mol. Biol., 222:581-597 (1991).

In the hybridoma method, a mouse or other appropriate host animal, such as a hamster or macaque monkey, is immunized as herein described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium. Human myeloma and mouse-human heteromyeloma cell lines also have been described for the production of human monoclonal antibodies (Kozbor, J. Immunol., 133: 3001 (1984) ;Brodeur et al., Monoclonal Antibody Production Techniques and Applications, pp. 51-63 (Marcel Dekker, Inc., New York, 1987)). Exemplary murine myeloma lines include those derived from MOP-21 and M.C.-11 mouse tumors available from the Salk Institute Cell Distribution Center, San Diego, Calif. USA, and SP-2 or X63-Ag8-653 cells available from the American Type Culture Collection, Rockville, Md. USA.

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The binding affinity of the monoclonal antibody can, for example, be determined by Scatchard analysis (Munson et al., Anal. Biochem., 107:220 (1980)).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

The term "antibody" is used in the broadest sense and includes fully assembled antibodies, monoclonal antibodies, polyclonal antibodies, multispecific antibodies (e.g., bispecific antibodies), antibody fragments that can bind antigen (e.g., Fab', F'(ab)2, Fv, single chain antibodies, diabodies), chimeric antibodies, humanized or human-derived antibodies, and recombinant peptides comprising the forgoing as long as they exhibit the desired biological activity.

Because chimeric or humanized antibodies are less immunogenic in humans than the parental mouse monoclonal antibodies, they can be used for the treatment of humans with far less risk of anaphylaxis. Thus, these antibodies may be preferred in therapeutic applications that involve in vivo administration to a human.

Chimeric monoclonal antibodies, in which the variable Ig domains of a mouse monoclonal antibody are fused to human constant Ig domains, can be generated using standard procedures known in the art (See Morrison, S. L., et al. (1984) Chimeric Human Antibody Molecules; Mouse Antigen Binding Domains with Human Constant Region Domains, Proc. Natl. Acad. Sci. USA 81, 6841-6855; and, Boulianne, G. L., et al, Nature 312, 643-646 . (1984)). Although some chimeric monoclonal antibodies have proved less immunogenic in humans, the mouse variable Ig domains can still lead to a significant human anti-mouse response.

Humanized antibodies may be achieved by a variety of methods including, for example: (1) grafting the non-human complementarity determining regions (CDRs) onto a human framework and constant region (a process referred to in the art as humanizing through "CDR grafting"), or, alternatively, (2) transplanting the entire non-human variable domains, but "cloaking" them with a human-like surface by replacement of surface residues (a process referred to in the art as "veneering"). In the present invention, humanized antibodies will include both "humanized" and "veneered" antibodies. These methods are disclosed in, e.g., Jones et al., Nature 321:522 525 (1986); Morrison et al., Proc. Natl. Acad. Sci., U.S.A., 81:6851 6855 (1984); Morrison and Oi, Adv. Immunol., 44:65 92 (1988); Verhoeyer et al., Science 239:1534 1536 (1988); Padlan, Molec. Immun. 28:489 498 (1991); Padlan, Molec. Immunol. 31(3):169 217 (1994); and Kettleborough, C.A. et al., Protein Eng. 4(7):773 83 (1991).

### Methods for detecting shM-CSFR

Disclosed herein is a method for detecting shM-CSFR in a biological sample comprising contacting the sample with an antibody that specifically binds to shM-CSFR and detecting either the antibody bound to shM-CSFR or the unbound antibody. The relative amounts of either correlate to the amount of shM-CSFR in the sample.

is directly or indirectly labeled with a detectable label.

Suitable detectable labels include various enzymes, binding groups, fluorescent materials, luminescent materials and radioactive materials. Exemplary enzyme labels include horseradish peroxidase, alkaline phosphatase, beta-galactosidase, or acetylcholinesterase; exemplary binding group complexes include streptavidin/biotin and avidin/biotin; exemplary fluorescent labels include umbelliferone, fluorescein, fluorescein isothiocyanate, rhodamine, dichlorotriazinylamine fluorescein, dansyl chloride or phycoerythrin; an exemplary luminescent label includes luminol; and exemplary radioactive labels include ¹²⁵I, ¹³¹I, ³⁵S or ³H.

In an RIA (radioimmunoassay), shM-CSFR can be detected by a competition immunoassay utilizing shM-CSFR standards linked to a detectable label and an unlabeled anti-shM-CSFR antibody. The biological sample, the labeled shM-CSFR standards and the anti-shM-CSFR antibody are combined and the amount of labeled anti-shM-CSFR standard bound to the unlabeled antibody is determined. The amount of shM-CSFR in the biological sample is inversely proportional to the amount of labeled shM-CSFR standard bound to the anti-shM-CSFR antibody.

An ELISA assay initially comprises preparing an antibody, specific to shM-CSFR, preferably a monoclonal antibody. In addition, a reporter antibody generally is prepared which binds specifically to shM-CSFR. The reporter antibody is attached to a detectable reagent such as radioactive, fluorescent or enzymatic reagent, for example horseradish peroxidase enzyme (HRP) or alkaline phosphatase (AP). To carry out the ELISA, antibody specific to shM-CSFR is incubated on a solid support, e.g. a polystyrene dish, that binds the antibody. Any free protein binding sites on the dish are then covered by incubating with a non-specific protein such as bovine serum albumin (BSA). Next, the sample to be analyzed is incubated in the dish, during which time shM-CSFR binds to the specific antibody attached to the polystyrene dish. Unbound sample is washed out with buffer. A reporter antibody specifically directed to shM-CSFR and linked to horseradish peroxidase is placed in the dish resulting in binding of the reporter antibody to any monoclonal antibody bound to shM-CSFR. Unattached reporter antibody is then washed out. Reagents for peroxidase activity, including a colorimetric substrate, are then added to the dish. Immobilized peroxidase, linked to anti-shM-CSFR antibodies, generate a colored reaction product. The amount of color developed in a given time period is proportional to the amount of shM-CSFR protein present in the sample. Quantitative results typically are obtained by reference to a standard curve.

These general assays can be utilized in a variety of formats, including the devices and kits described below.

### Devices and kits for detecting shM-CSFR

The assay device may be a a lateral flow test strip, optionally encased in a housing. A first labeled antibody to shM-CSFR is in solution, while a second antibody to shM-CSFR is immobilized on the test strip. When a patient sample containing shM-CSFR is contacted with both antibodies, an antibody-target-antibody sandwich complex is formed, and the resulting complex, which is immobilized on the solid support, is detectable by virtue of the label. The test strip is then inserted into a reader, where the signal from the label in the complex is measured. The outcome may be either a positive or negative result, or a quantitative determination of the concentration of shM-CSFR in the sample, which is correlated with a result indicative of a risk or presence of a disease or disorder. The entire procedure may be automated and/or computer-controlled. Alternatively, the test strip may be read visually by comparison to a visual standard of the appropriate color. This test provides similar clinically relevant information as a shM-CSFR ELISA, but in significantly less time and at the point of care.

The device may be a test strip for use in the rapid detection of shM-CSFR, using immunoassay methods in which shM-CSFR in a fluid sample compete with immobilized shM-CSFR for limited labeled antibody binding sites. In the assay procedure, the body fluid sample mixes with labeled antibody-dye conjugate and migrates along a porous membrane. When the concentration of shM-CSFR is below the detection limit of the test, unbound antibody-dye conjugate binds to shM-CSFR conjugate immobilized on the membrane, producing a color band in the "negative" test zone. Conversely, when the shM-CSFR level is at or above the detection limit, free shM-CSFR competes with the immobilized shM-CSFR conjugate on the membrane by binding to antibody-dye conjugate, forming an antigen- antibody complex, and thus preventing the development of a color band. Regardless of the shM-CSFR levels in the sample, a color band is produced in each control zone, which serves as a quality control measures that verifies that the reagents are chemically active.

Similar methods can be used for more quantitative determinations of shM-CSFR level, based on gradations in color intensity.

Methods of setting a threshold by which disease states, prognoses and the like are measured are well known in the art. By way of example, levels of shM-CSFR protein in a fluid sample from a sufficient representative number of normal subjects (e.g. healthy population without the condition to be detected) are analyzed relative to the shM-CSFR protein level from a sufficient representative number of diseased subjects (e.g. population confirmed to have the disease or condition). A threshold cutoff can be determined that differentiates most of the normal population from most the diseased population. Alternatively, useful end point values for negative, uncertain and positive results can be determined from the data. For example, a normal range (indicative of a negative result) can be determined, which includes shM-CSFR levels of most of the normal population but which exclude almost all of the diseased population. Correspondingly, a range indicative of a positive result can be determined, which includes shM-CSFR levels of most of the diseased population but which exclude almost all of the normal population.

Measured levels of shM-CSFR protein either above (or below, as appropriate) the predetermined threshold are indicative of an association of shM-CSFR protein with the disease. In order to make use of the threshold, samples from the normal population should be subjected to identical protocols (i.e., preparation and storage) as the samples from the unhealthy population. Looking at the range of measured shM-CSFR protein levels from samples from the normal population, it will be apparent to the skilled worker as to the appropriate threshold level and desired specificity or sensitivity for making comparisons, prognoses, and diagnoses considering the range of measured shM-CSFR protein levels from samples from the unhealthy population.

Ideally, determination of such thresholds take account of overall medical and epidemiological factors. Factors to be considered include the clinical objective of the laboratory test and whether it is necessary to have a high positive predictive value, or a high negative predictive value, as well as prevalence of the disease in the test population.

Kits are also contemplated. A typical kit can comprise a first antibody that specifically binds to shM-CSFR, optionally linked to a detectable label, and an M-CSFR standard containing a known quantity of M-CSFR. Other components may optionally include reagents for carrying out an immunoassay such as a second antibody linked to a detectable label that either binds to shM-CSFR or to the first antibody; if the label is an enzyme, the kit may also include a substrate from which the enzyme releases a detectable signal.

### Combination Therapy

It may be advantageous to mix two or more M-CSF antagonists (e.g., shM-CSFR and an anti-M-CSF antibody) together to provide still improved efficacy against diseases or disorders associated with M-CSF (e.g., cancer, cancer metastasis and/or osteolysis). Compositions comprising one or more M-CSF antagonists may be administered to persons or mammals suffering from, or predisposed to suffer from, cancer metastasis and/or bone loss associated with cancer metastasis. Concurrent administration of two therapeutic agents does not require that the agents be administered at the same time or by the same route, as long as there is an overlap in the time period during which the agents are exerting their therapeutic effect. Simultaneous or sequential administration is contemplated, as is administration on different days or weeks. Combining the shM-CSFR therapy method with a chemotherapeutic or radiation regimen may also be carried out.

### Administration and preparation

The shM-CSFR used may be formulated into a pharmaceutical composition comprising a carrier suitable for the desired delivery method. Suitable carriers include any material which, when combined with the shM-CSFR, retains the anti-tumor function of the shM-CSFR and is non-reactive with the subject's immune systems. Examples include, but are not limited to, any of a number of standard pharmaceutical carriers such as sterile phosphate buffered saline solutions, bacteriostatic water, and the like. A variety of aqueous carriers may be used, e.g., water, buffered water, 0.4% saline, 0.3% glycine and the like, and may include other proteins for enhanced stability, such as albumin, lipoprotein, globulin, etc., subjected to mild chemical modifications or the like.

Therapeutic formulations of the drug are prepared for storage by mixing the drug having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

Compositions disclosed herein are administered to a mammal in an amount sufficient to prevent or at least partially arrest the progression of cancer, cancer metastasis and/or osteolysis. An amount adequate to accomplish this is defined as a "therapeutically effective dose." Single or multiple administrations of the compositions can be carried out with the dose levels and pattern being selected by the treating physician. For the prevention or treatment of disease, the appropriate dosage will depend on the type of disease to be treated, as defined above, the severity and course of the disease, whether drug is administered for preventive or therapeutic purposes, previous therapy, the patient's clinical history and response to the drug, and the discretion of the attending physician.

### Non-therapeutic uses

The shM-CSFR disclosed herein may also be used as affinity purification agent for M-CSF or in diagnostic assays for M-CSF protein, e.g., detecting its expression in specific cells, tissues, or serum. The shM-CSFR may also be used for *in vivo* diagnostic assays. Generally, for these purposes the shM-CSFR is labeled with a radionuclide (such as ¹¹¹In, ⁹⁹Tc, ¹⁴C, ¹³¹I, ¹²⁵I, ³H, ³²P or ³⁵S) so that the tumor can be localized using immunoscintiography.

The shM-CSFR disclosed herein may be employed in any known assay method, such as competitive binding assays, direct and indirect sandwich assays, such as ELISAs, and immunoprecipitation assays. Zola, Monoclonal Antibodies: A Manual of Techniques, pp.147-158 (CRC Press, Inc. 1987).

As a matter of convenience, the shM-CSFR disclosed herein when used to detect M-CSF can be provided in a kit, i.e., a packaged combination of reagents in predetermined amounts with instructions for performing the diagnostic assay, optionally including an M-CSF standard containing a known quantity of M-CSF. In addition, other additives may be included such as stabilizers, buffers (e.g., a block buffer or lysis buffer) and the like. The relative amounts of the various reagents may be varied widely to provide for concentrations in solution of the reagents which substantially optimize the sensitivity of the assay. Particularly, the reagents may be provided as dry powders, usually lyophilized, including excipients which on dissolution will provide a reagent solution having the appropriate concentration.

The invention is illustrated by the following examples, which are not intended to be limiting in any way.

### EXAMPLES

### EXAMPLE 1

This example describes the identification of a soluble receptor for human M-CSF and shows that this soluble receptor is capable of binding M-CSF.

### I. Identification of soluble human M-CSF receptor in serum samples

### A. Materials and Methods

Micro titer plates (R&D systems, Cat# CP0011) were coated with 100 µl/well capture antibody (Duo Set Elisa development system hM-CSF R (R&D systems, Cat# DY329)) at a working dilution of 100 ng/ml in PBS. The plates were sealed and incubated at room temperature overnight. After washing the wells 3 times with wash buffer (0.05% Tween in PBS, pH 7.2-7.4) with a manifold dispenser/washer, plates were blocked with 300 µl/well of blocking buffer (1% BSA, 5% Sucrose in PBS, pH 7.2-7.4) at room temperature for at least 1 hour.

After repeating the wash step, the plates were ready for sample addition. 100 µl/well of either sample or standards in reagent diluent were added. Plates were covered with adhesive and incubated for 2 hours at room temperature, followed by 3 wash cycles. After adding 100 µl of detection antibody (Duo Set Elisa development system h M-CSF R (R&D systems, Cat# DY329)), the plates were covered and incubated for 2 hours at room temperature followed by 3 wash cycles.

Next, 100 µl of the working dilution of Streptadvidin HRP (Duo Set Elisa development system hM-CSFR (R&D systems, Cat# DY329)) were added followed by an incubation of 20 minutes at room temperature in the dark. After 3 wash cycles, the plates were developed for 5 minutes with 100 µl substrate solution (1:1 Mixture of Reagent A and B (R&D systems, Cat# DY999)) per well, followed by addition of 50 µl stop solution (2N H₂SO₄), and gentle tapping of the plate to ensure thorough mixing. The final readout was the optical density at 450-540 nm immediately after addition of stop solution.

### B. Results

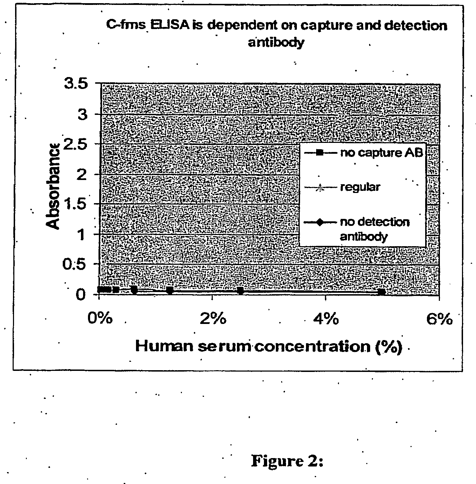

A strong positive signal was detected in human serum samples when the ELISA assay was used to determine the presence of soluble human M-CSF receptor in serum samples. Both the capture antibody and detection antibody used in this ELISA assay were specific for human M-CSF receptor extra-cellular domain. As shown in Figure 2, a strong positive signal was detected in human serum samples. The signal is dependent on the concentration of human serum. There is no signal when no capture antibody was used.

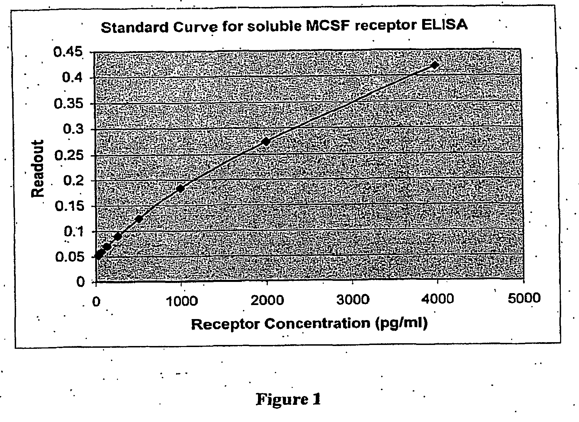

A standard curve was established to quantify the concentration of soluble human M-CSF receptor, using a recombinant human M-CSF receptor and human antibody Fc fragment fusion protein as reference protein (Figure 1). Using this ELISA assay, the amount of the soluble receptor in human serum was calculated with the c-fms human Fc fusion protein as reference. It was found that about 0.5 ug/ml soluble human M-CSF receptor is present in the human serum (Figure 2).

### II. ELISA binding assay for soluble M-CSF receptor

### A. Materials and Methods

Micro titer plates (R&D systems, Cat# CP0011) were coated with 100 µl /well capture antibody (Duo Set Elisa development system hM-CSF R (R&D systems, Cat# DY329)) at a working dilution of 100 ng/ml in PBS. The plates were sealed and incubated at room temperature overnight. After washing the wells 3 times with wash buffer (0.05% Tween in PBS, pH 7.2-7.4) with manifold dispenser/washer, plates were blocked with 300 µl/well blocking buffer (1% BSA, 5% Sucrose in PBS, pH 7.2-7.4) at room temperature for at least 1 hour.

After repeating the wash step, plates were ready for sample addition. 100 µl/well of sample or standards in reagent diluent were added. Plates were covered with adhesive and incubated for 2 hours at room temperature, followed by 3 wash cycles. Human M-CSF at 1 µg/mL was added to the plate and incubated at room temperature for 1 hour. After adding 100 µl of detection antibody against human M-CSF (HRP-conjugated polyclonal anti-human M-CSF antibody: R&D Systems, Cat DMC00, part#890154), the plates were covered and incubated for 2 hours at room temperature, followed by 3 wash cycles. Plates were developed for 5 minutes with 100 µl substrate solution (1:1 Mixture of Reagent A and B (R&D systems, Cat# DY999)) per well, followed by addition of 50 µl stop solution (2N H₂SO₄), gently tapping the plate to ensure thorough mixing. The final readout was the optical density at 450-540 nm immediately after addition of stop solution.

### B. Results

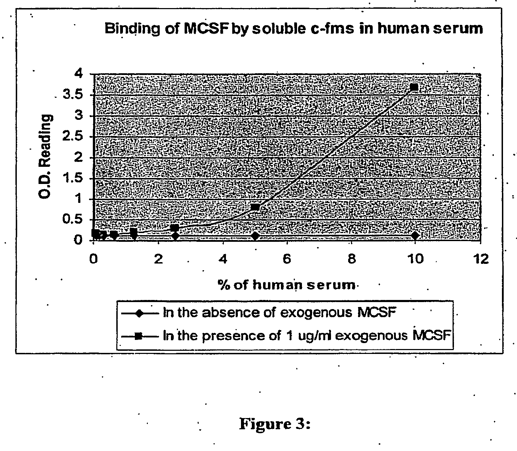

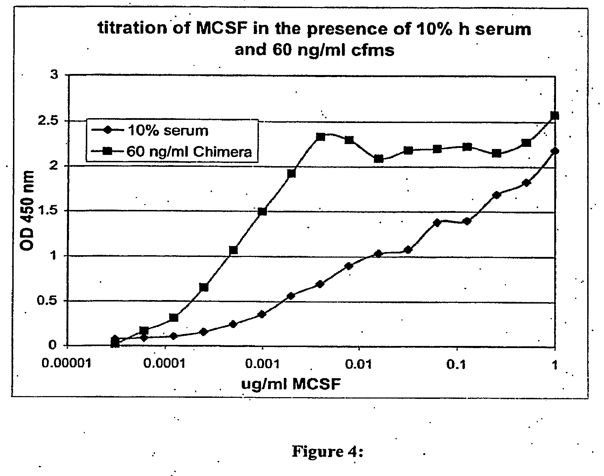

The soluble receptor present in human serum is capable of binding M-CSF. As shown in Figure 3, a serial dilution of human serum sample was applied to an ELISA plate coated with a mouse monoclonal anti-human c-fins antibody. Either exogenous human M-CSF at 1 µg/ml (squares) or PBS (diamonds) was added before detection of human M-CSF with an alkaline phosphatase-conjugated polyclonal anti-human M-CSF antibody. Distinct binding of M-CSF was found above 5% human serum. However, the soluble receptor in human serum was found to have lower binding affinity as compared with recombinant c-fins Fc fusion protein (Figure 4) [The binding affinity of the recombinant c-fms Fc fusion protein is considered to be close to that of the native membrane-bound c-fms because of the ligand-induced dimerization]. One interpretation for the low affinity of the soluble receptor is that it has only single binding site for the ligand, while the recombinant c-fins Fc fusion protein has two binding sites for avidity effect.

### III. Immunoprecipitation of human M-CSF receptor from serum samples

### A. Materials and Methods

500 µl Streptavidin beads (#20347, Pierce) were added to 10 ml 20% human serum (Sigma Cat. # S 7023, previously frozen) in 15 ml Falcon tube and incubated for 1 hour at 4°C, with gentle rocking. The sample was spun for 5 minutes at 400 rpm and the supernatant transferred to a fresh tube. 10 µg anti-hMCSFR antibody (R&D # BAF 329) was added to the supernatant and incubated for 1 hour at 4°C, with gentle rocking. After adding 500 µl Streptavidin beads, the sample was incubated at 4°C with rocking overnight. The sample was spun for 5 minutes at 400 rpm and the supernatant was transferred to a fresh tube, and the supernatant was saved at the same time. The beads were transferred to an Eppendorf tube and washed once with 1 ml each of wash buffers #1(0.5 M LiCL₂), #2(0.5 M LiCL₂/ 0.5% Triton) and #3(10 mM TRIS pH 7.4). After the last spin, most of the wash buffer was removed (until the beads were almost dry). The beads were then re-suspended in 500 µl PBS. Before running the gel (10% Novex), 5x SDS sample buffer with DTT was added, and the samples were boiled for 2 minutes at 100°C.

In order to deglycosylate the immunoprecipatated human M-CSF receptor, frozen beads from the above immunoprecipitation procedure ( ~ 200 µl) were diluted with 200 µl PBS at a 1:1 ratio. The diluted sample was incubated in 1x denaturation buffer (PNGase F, NEB# P0705S) at 100°C for 10 minutes. After addition of G7 reaction buffer (PNGase F, NEB # P0705S) to 1x final concentration and NP40 (final conc. 1 %) the sample was incubated at 37°C for 1 hour with 5 µl PNGase F.

### B. Results

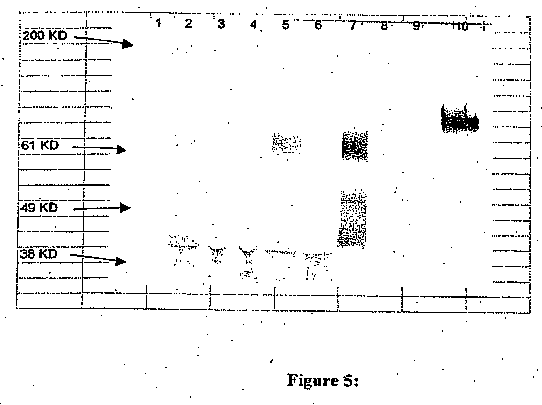

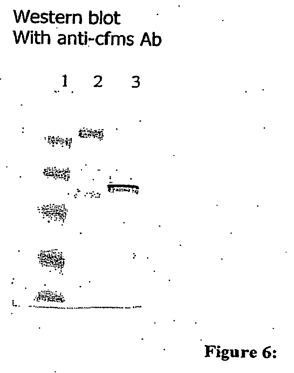

The soluble human M-CSF receptor was found to be a glycosylated protein with a molecular weight around 97 KD (Figure 5). After deglycosylation, the molecular weight of the soluble human M-CSF receptor is about 60 KD (Figure 6).

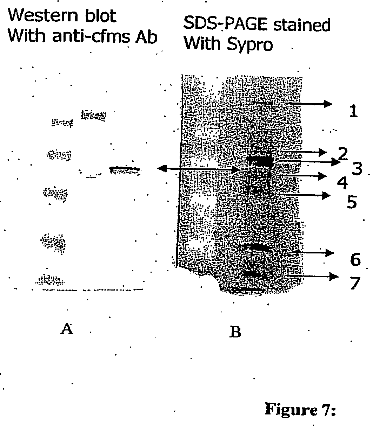

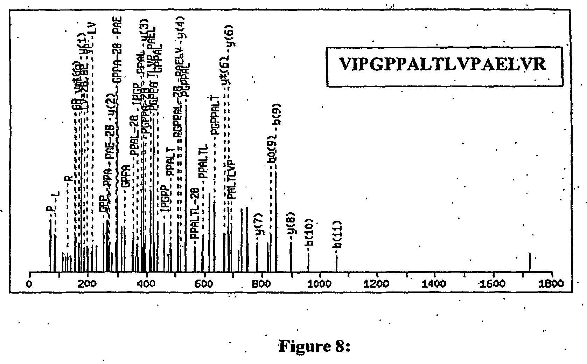

The soluble human M-CSF receptor was found to share, at least partially, the protein sequence of the human c-fms protein, which is the membrane-bound receptor for human M-CSF. As shown in above figures, the soluble human M-CSF receptor can be recognized by multiple antibodies that also recognizes human c-fins protein. To further confirm the protein sequence of the soluble human M-CSF receptor, a protein analysis was performed as follows: A protein band was identified on a SDS-PAGE gel (band #4 in Figure 7) that corresponded to the soluble receptor after deglycosylation. The protein in this band was digested with trypsin. The product of this digestion, a mixture of peptides, was analyzed. One peptide was found to have the exact molecular weight and amino acid sequence (SEQ ID NO: 1) as a segment in the extracellular domain of human c-fms protein (Figure 8). This evidence indicates that the soluble human M-CSF receptor shares the protein sequence of the human c-fms protein, which is the membrane-bound receptor for human M-CSF.

### EXAMPLE 2

This Example shows that the soluble M-CSF receptor was found in human urine samples. This Example further shows that this receptor is present in serum from both normal subjects and breast cancer patients, and the soluble M-CSF receptor level correlated with the M-CSF level. Finally, this Example shows that the soluble M-CSF receptor is also found in primates.

### I. Soluble human M-CSF receptor was also found in urine samples

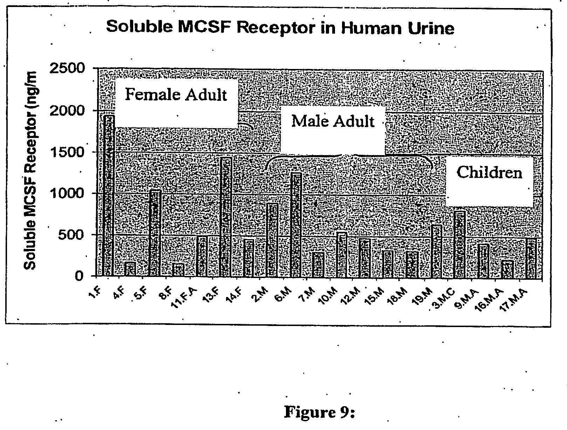

Using the same ELISA setup as described in Example 1, multiple urine samples from healthy human volunteers were collected and assayed for the presence of soluble human M-CSF receptor. Varying levels of soluble human M-CSF receptor were detected among adult female (age from 25 to 66), adult male (age from 35 to 70) and children from age 3 to 9. Quantification of the concentration of soluble human M-CSF receptor was determined with recombinant c-fms-human Fc fusion protein as reference (Figure 9).

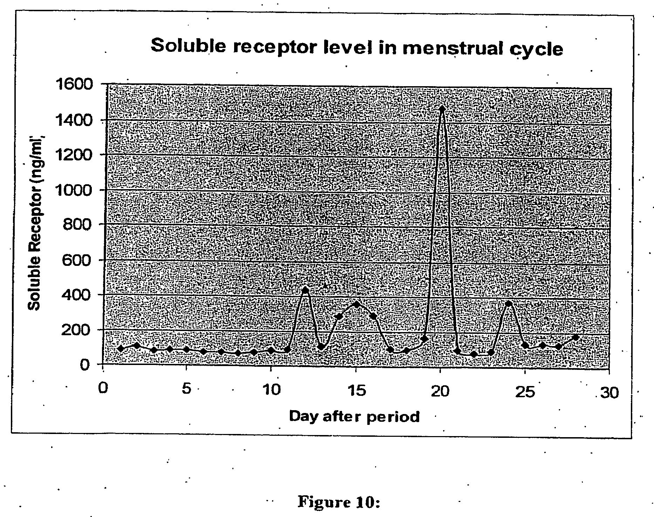

Urine samples were collected daily throughout the menstrual cycle of one female subject. The samples were collected as first void in the morning and frozen at 20°C. All samples were assayed on the same date. Soluble receptor was assayed as described herein. As shown in Figure 10, soluble receptor level urine samples fluctuates during the menstrual cycle. This finding makes the soluble receptor a potential diagnostic marker for the menstrual cycle, or for use in pregnancy tests.

### II. Correlation of soluble M-CSF receptor level with M-CSF level in breast cancer patients

Soluble human M-CSF receptor concentrations were analyzed in breast cancer serum samples as follows. A serial dilution of human serum sample was assayed in an ELISA for the presence of soluble human M-CSF receptor as described above. A strong positive signal was detected when the human serum was assayed in this ELISA format (referred to as "regular"). As controls, removal of either the capture antibody or detection antibody eliminated the signal, indicating that the signal is specific for these anti-human c-fms antibodies. Quantification of the concentration of soluble human M-CSF receptor was determined to be 0.5 ug/ml, with recombinant c-fms-human Fc fusion protein as reference.

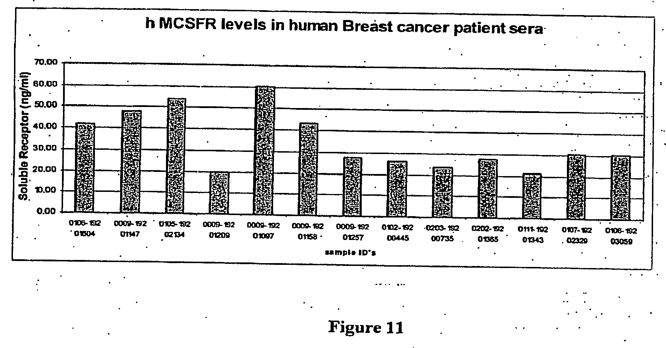

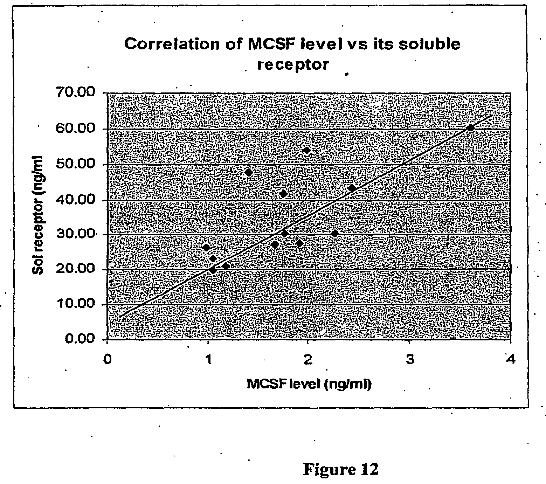

As shown in Figure 11, the level of soluble receptor varies among patients. It is noted that the soluble receptor concentrations in the patients' samples are much lower than described above (Figure 2). likely due to different protocols used for serum sample preparation and storage. Consequently, the comparison of M-CSF and M-CSF soluble receptor concentration is valid only among samples collected and treated according to the same protocol. Figure 12 shows a linear correlation between M-CSF levels in the breast cancer patients versus M-CSF soluble receptor concentration in the same sample. This finding makes M-CSF soluble receptor a potential biomarker for cancer progression. Moreover, M-CSF soluble receptor becomes a more useful biomarker when direct measurement of M-CSF becomes difficult (e.g., when a M-CSF neutralizing antibody is used as a treatment of cancer).

### III. Soluble M-CSF receptor also found in other primates

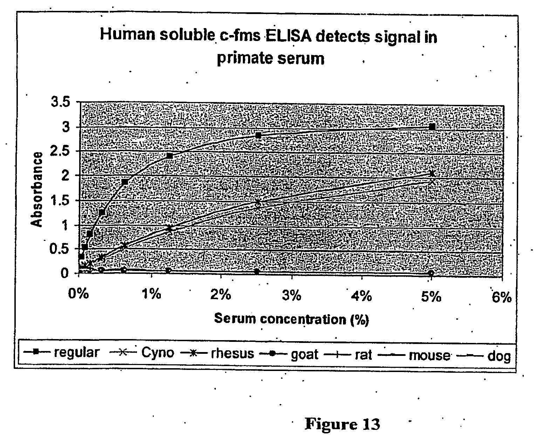

The ELISA assay described above for human M-CSF soluble receptor was also used to test serum samples from other animal species (Figure 13). Cynomulgus monkey and Rhesus monkey serum samples show a strong positive signal, indicating the presence of soluble receptors. The protein sequences of the membrane-bound M-CSF receptor for Cyno and Rhesus monkeys are not available. However, it is believed that they share a high level of protein homology with their human counterpart. Therefore, the antibodies used in the ELISA assay, which recognize the extracellular domain of human membrane-bound M-CSF receptor, also recognize that of the Cyno and Rhesus monkeys.

It should be noted that the lack of positive signal from non-primate animal serum does not indicate the lack of M-CSF soluble receptor. Rather, it is most likely due to the antibody specificity of the ELISA assay used to detect the presence of soluble receptor.

### EXAMPLE 3

This Example shows that the expression of soluble human M-CSF receptor is tightly coupled with osteoclast differentiation. This Example further shows that only differentiated osteoclasts express the soluble human M-CSF receptor, the level of which increases upon removal of M-CSF.

### A. Materials and Methods

The expression of M-CSF soluble receptor was studied in an in vitro system with human osteoclast cells, where the expression of the membrane-bound M-CSF receptor was found. In particular, the expression of the soluble receptor was examined in the context of osteoclast differentiation. Primary human Osteoclast Precursors (Cambrex Bio Science Walkersville, Inc. Cat# 2T-110) were seeded at 10,000 cells/well at 0.2 ml/well in the cell culture media containing 30 ng/ml human M-CSF and 100 ng/ml RANKL. Test antibodies were added to the wells at 1 µg/mL on the same day of cell plating. The cells were incubated at 37°C, in a humidified atmosphere of 5% CO₂. On day 7, osteoclasts were identified by phase microscopy as unusually large multinucleate cells. The culture can be continued for an additional week, with feeding, during which time the osteoclasts will continue to increase in size. At the end of the culture, cells were stained for tartrate-resistant acid phosphatase, the positive staining of which indicates the presence of osteoclasts. The number of osteoclasts in each well was counted under the light microscope and shown in the graph as an average of osteoclasts under the viewing field of the microscope.

TRAP activity in osteoclast conditioned media was assayed with BoneTRAP Assay kit (TR201) from SBA-Sciences. BoneTRAP® Assay (TR201) is a solid-phase immunofixed enzyme activity assay for the rapid and specific determination of bone resorption rate from human serum samples. It can be used in human osteoclast cultures.

### B. Results

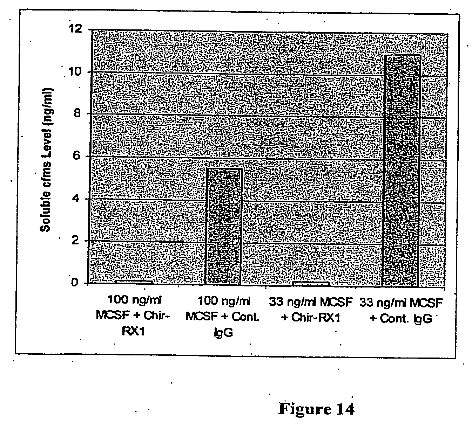

As shown in Figure 14, the M-CSF soluble receptor was expressed in osteoclast cells. The concentration of the soluble receptor in the conditioned media of differentiated osteoclasts was measured. The expression of soluble receptor was specific to osteoclasts. Inhibition of osteoclast differentiation by a M-CSF neutralizing antibody, Chir-RX1 in this case, resulted in the suppression of soluble receptor.

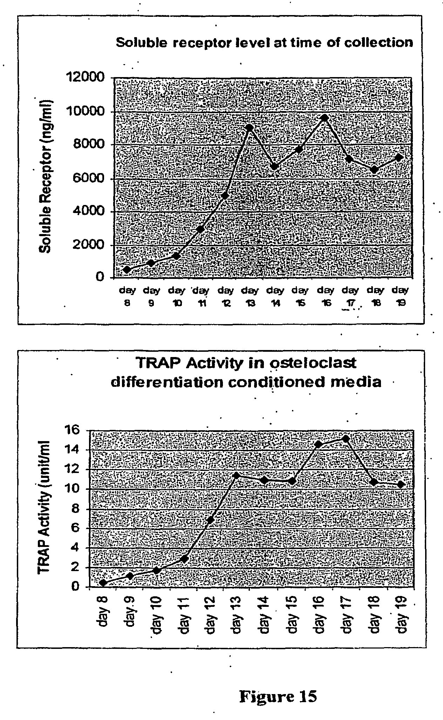

Soluble receptor is expressed only in differentiated osteoclasts, not osteoclast precursors. As shown by a time course experiment shown in Figure 15a, soluble receptor expression starts on Day 10 in the osteoclast differentiation assay and reaches plateau on Day 12. This expression pattern parallels exactly that of osteoclast differentiation, as followed by TRAP activity and shown in Figure 15b.

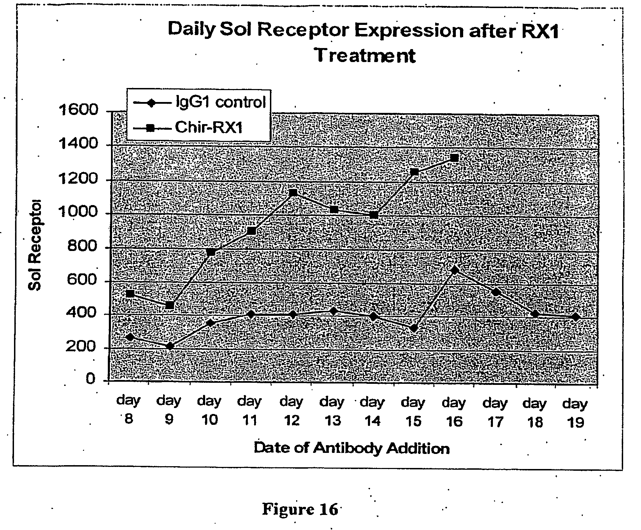

Interestingly, M-CSF soluble receptor expression is up-regulated by the removal of M-CSF in differentiated osteoclasts. Figure 16 shows the expression of M-CSF soluble receptor within 24 hours after the addition of either an IgG1 control antibody or M-CSF neutralizing antibody, Chir-RX1. The data clearly show that soluble receptor expression goes up when M-CSF activity is neutralized. The increase of expression starts at Day 8 and reaches the highest level on Day 12, which indicates that the increased expression happens in differentiated osteoclasts.

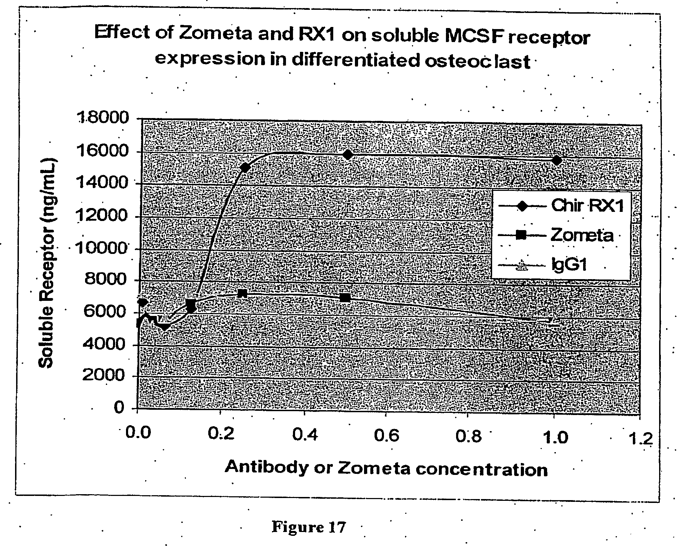

Zometa, an inhibitor of osteoclast activity, has no effect on the expression of M-CSF soluble receptor in differentiated osteoclasts (Figure 17). In the same experiment, M-CSF neutralizing antibody, not the IgG1 control, stimulated the expression.

## Claims

1. An *in vitro* method of diagnosing breast cancer comprising the step of:
(a) analyzing a fluid sample from a patient for level of soluble M-CSF receptor, wherein a level of soluble M-CSF receptor above a threshold is correlated with the presence of breast cancer and a level below said threshold indicates that the patient is unlikely to have cancer.

2. An *in vitro* method of determining prognosis in a subject afflicted with breast cancer comprising the step of:
(a) analyzing a fluid sample from a patient for level of soluble M-CSF receptor, wherein a level of soluble M-CSF receptor above a threshold indicates that the patient is likely to have a poor prognosis and a level below said threshold indicates that the patient is likely to have a good prognosis.

3. An *in vitro* method of monitoring breast cancer therapy in a subject afflicted with breast cancer comprising the steps of:
(a) analyzing a fluid sample from a patient for level of soluble M-CSF receptor prior to the initiation of treatment with a cancer therapeutic; and
(b) analyzing said fluid sample after the initiation of the treatment with the cancer therapeutic,
wherein a reduction in the level of soluble M-CSF receptor after the initiation of the treatment with the cancer therapeutic indicates the patient is receiving a therapeutically effective dose of the cancer therapeutic.

4. The method according to any one of the preceding claims wherein the fluid sample is selected from the group consisting of urine, plasma, or serum.

5. Use of a kit in a method according to any one of claims 1-4, the kit comprising:
(a) a first antibody that specifically binds in a fluid sample to shM-CSFR; and
(b) an M-CSFR standard containing a known quantity of M-CSFR.

6. The use of claim 5 wherein said first antibody is linked to a detectable label.

7. The use of claim 6 wherein said label is an enzyme.

8. The use of claim 7 further comprising a substrate from which said enzyme releases a detectable signal.

9. The use of claim 5 further comprising a second antibody that binds to shM-CSFR.

10. The use of claim 5 further comprising a second antibody that binds to said first antibody.

11. The use of claim 5 further comprising a second antibody that binds to shM-CSFR.

## Patentansprüche

1. In-vitro-Verfahren zur Diagnose von Brustkrebs, umfassend den Schritt:
(a) Analysieren einer Flüssigkeitsprobe von einem Patienten auf den Spiegel von löslichem M-CSF-Rezeptor, wobei ein Spiegel von löslichem M-CSF-Rezeptor oberhalb eines Schwellenwerts mit dem Vorliegen von Brustkrebs korreliert ist und ein Spiegel unterhalb des Schwellenwerts anzeigt, dass bei dem Patienten wahrscheinlich kein Krebs vorliegt.

2. In-vitro-Verfahren zur Bestimmung einer Prognose bei einem von Brustkrebs betroffenen Individuum, umfassend den Schritt:
(a) Analysieren einer Flüssigkeitsprobe von einem Patienten auf den Spiegel von löslichem M-CSF-Rezeptor, wobei ein Spiegel von löslichem M-CSF-Rezeptor oberhalb eines Schwellenwerts anzeigt, dass die Prognose für den Patienten wahrscheinlich schlecht ist, und ein Spiegel unterhalb des Schwellenwerts anzeigt, dass die Prognose für den Patienten wahrscheinlich gut ist.

3. In-vitro-Verfahren zur Überwachung einer Brustkrebstherapie bei einem von Brustkrebs betroffenen Individuum, umfassend die Schritte:
(a) Analysieren einer Flüssigkeitsprobe von einem Patienten auf den Spiegel von löslichem M-CSF-Rezeptor vor Beginn einer Behandlung mit einem Krebstherapeutikum; und
(b) Analysieren der Flüssigkeitsprobe nach Beginn der Behandlung mit dem Krebstherapeutikum,
wobei eine Verringerung des Spiegels von löslichem M-CSF-Rezeptor nach Beginn der Behandlung mit dem Krebstherapeutikum anzeigt, dass der Patient eine therapeutisch wirksame Dosis des Krebstherapeutikums erhält.

4. Verfahren nach einem der vorhergehenden Ansprüche, wobei die Flüssigkeitsprobe aus der aus Harn, Plasma oder Serum bestehenden Gruppe ausgewählt ist.

5. Verwendung eines Kits bei einem Verfahren gemäß einem der Ansprüche 1-4, wobei das Kit Folgendes umfasst:
(a) einen ersten Antikörper, der in einer Flüssigkeitsprobe spezifisch an shM-CSFR bindet; und
(b) einen M-CSFR-Standard, der eine bekannte Menge an M-CSFR enthält.

6. Verwendung nach Anspruch 5, wobei der erste Antikörper mit einer nachweisbaren Markierung verknüpft ist.

7. Verwendung nach Anspruch 6, wobei es sich bei der Markierung um ein Enzym handelt.

8. Verwendung nach Anspruch 7, ferner umfassend ein Substrat, aus dem das Enzym ein nachweisbares Signal freisetzt.

9. Verwendung nach Anspruch 5, ferner umfassend einen zweiten Antikörper, der an shM-CSFR bindet.

10. Verwendung nach Anspruch 5, ferner umfassend einen zweiten Antikörper, der an den ersten Antikörper bindet.

11. Verwendung nach Anspruch 5, ferner umfassend einen zweiten Antikörper, der an shM-CSFR bindet.

## Revendications

1. Procédé *in vitro* de diagnostic du cancer du sein comprenant l'étape consistant :
(a) à analyser un échantillon de fluide prélevé chez une patiente pour déterminer le niveau de récepteur soluble du facteur stimulant la prolifération des macrophages (M-CSF), dans lequel un niveau de récepteur soluble du M-CSF supérieur à un seuil est corrélé à la présence d'un cancer du sein, tandis qu'un niveau inférieur audit seuil indique qu'il est peu probable que la patiente souffre d'un cancer.

2. Procédé *in vitro* de détermination du pronostic chez un sujet souffrant d'un cancer du sein comprenant l'étape consistant :
(a) à analyser un échantillon de fluide prélevé chez une patiente pour déterminer le niveau de récepteur soluble du M-CSF, dans lequel un niveau de récepteur soluble du M-CSF supérieur à un seuil indique qu'il est probable que la patiente présente un mauvais pronostic, tandis qu'un niveau inférieur audit seuil indique qu'il est probable que la patiente présente un bon pronostic.

3. Procédé *in vitro* de suivi du traitement d'un cancer du sein chez un sujet souffrant d'un cancer du sein comprenant les étapes consistant :
(a) à analyser un échantillon de fluide prélevé chez une patiente pour déterminer le niveau de récepteur soluble du M-CSF avant la mise en place du traitement par un agent thérapeutique anticancéreux ; et
(b) à analyser ledit échantillon de fluide après la mise en place du traitement par l'agent thérapeutique anticancéreux,
dans lequel une réduction du niveau de récepteur soluble du M-CSF après la mise en place du traitement par l'agent thérapeutique anticancéreux indique que la patiente reçoit une dose thérapeutiquement efficace d'agent thérapeutique anticancéreux.

4. Procédé selon l'une quelconque des revendications précédentes dans lequel l'échantillon de fluide est choisi dans le groupe constitué de l'urine, du plasma ou du sérum.

5. Utilisation d'un kit dans un procédé selon l'une quelconque des revendications 1 à 4, le kit comprenant :
(a) un premier anticorps qui se lie de façon spécifique dans un échantillon de fluide au récepteur soluble humain du M-CSF (shM-CSFR) ; et
(b) un étalon M-CSFR contenant une quantité connue de M-CSFR.

6. Utilisation selon la revendication 5 dans laquelle ledit premier anticorps est lié à un marqueur détectable.

7. Utilisation selon la revendication 6 dans laquelle ledit marqueur est une enzyme.

8. Utilisation selon la revendication 7 comprenant, en outre, un substrat depuis lequel ladite enzyme émet un signal détectable.

9. Utilisation selon la revendication 5 comprenant, en outre, un second anticorps qui se lie au shM-CSFR.

10. Utilisation selon la revendication 5 comprenant, en outre, un second anticorps qui se lie audit premier anticorps.

11. Utilisation selon la revendication 5 comprenant, en outre, un second anticorps qui se lie au shM-CSFR.


### SEQUENCE LISTING

<110> Liu et al., Cheng
<120> Soluble Human M-CSF Receptor and Uses Thereof
<130> PP028096.0003 (27527/41256/PCT)
<160> 2
<170> Patent In version 3.3
<210> 1
   <211> 17
   <212> PRT
   <213> homo sapiens
<400> 1 Arg
<210> 2
   <211> 957
   <212> PRT
   <213> homo sapiens
<400> 2
   Met Gly Pro Gly Val Leu Leu Leu Leu Leu Val Ala Thr Ala Trp His
   1               5                   10                  15
   Gly Gln Gly Ile Pro Val Ile Glu Pro Ser Val Pro Glu Leu Val Val
               20                  25                  30
   Lys Pro Gly Ala Thr Val Thr Leu Arg Cys Val Gly Asn Gly Ser Val
           35                  40                  45
   Glu Trp Asp Gly Pro Pro Ser Pro His Trp Thr Leu Tyr Ser Asp Gly
       50                  55                  60
   Ser Ser Ser Ile Leu Ser Thr Asn Asn Ala Thr Phe Gln Asn Thr Gly
   65                  70                  75                  80
   Cys Thr Glu Pro Gly Asp Pro Leu Gly Gly Ser Ala Ala Ile His Leu
                   85                  90                  95
   Tyr Val Lys Asp Pro Ala Arg Pro Trp Asn Val Leu Ala Gln Glu Val
               100                 105                 110
   Val Val Phe Glu Asp Gln Asp Ala Leu Leu Pro Cys Leu Leu Thr Asp
   115 120 125
   Pro Val Leu Glu Ala Gly Val Ser Leu Val Arg Val Arg Gly Arg Pro
   130 135 140
   Leu Met Arg His Thr Asn Tyr Ser Phe Ser Pro Trp His Gly Phe Thr
   145 150 155 160
   Ile His Arg Ala Lys Phe Ile Gln Ser Gln Asp Tyr Gln Cys Ser Ala
   165 170 175
   Leu Met Gly Gly Arg Lys Val Met Ser Ile Ser Ile Arg Leu Lys Val
   180 185 190
   Gln Lys Val Ile Pro Gly Pro Pro Ala Leu Thr Leu Val Pro Ala Glu
   195 200 205
   Leu Val Arg Ile Arg Gly Glu Ala Ala Gln Ile Val Cys Ser Ala Ser
   210 215 220
   Ser Val Asp Val Asn Phe Asp Val Phe Leu Gln His Asn Asn Thr Lys
   225 230 235 240
   Leu Ala Ile Pro Gln Gln Ser Asp Phe His Asn Asn Arg Tyr Gln Lys
   245 250 255
   Val Leu Thr Leu Asn Leu Asp Gln Val Asp Phe Gln His Ala Gly Asn
   260 265 270
   Tyr Ser Cys Val Val Gln Gly Lys His Ser Thr Ser Met Phe Phe Arg
   275 280 285
   Val Val Glu Ser Ala Tyr Leu Asn Leu Ser Ser Glu Gln Asn Leu Ile
   290 295 300
   Gln Glu Val Thr Val Gly Glu Leu Lys Val Met Val Glu Ala Tyr Pro
   305 310 315 320
   Gly Leu Gln Gly Phe Asn Trp Thr Tyr Leu Gly Pro Phe Ser Asp His
   325 330 335
   Gln Pro Glu Pro Lys Leu Ala Asn Ala Thr Thr Lys Asp Thr Tyr Arg
   340 345 350
   His Thr Phe Thr Leu Ser Leu Pro Arg Leu Lys Pro Ser Glu Ala Gly
   355 360 365
   Arg Tyr Ser Phe Leu Ala Arg Asn Pro Gly Gly Trp Arg Ala Leu Thr
   370 375 380
   Phe Glu Leu Thr Leu Arg Tyr Pro Pro Glu Val Ser Val Ile Trp Thr
   385 390 395 400
   Phe Ile Asn Gly Ser Gly Thr Leu Leu Cys Ala Ala Ser Gly Tyr Pro
   405 410 415
   Gln Pro Asn Val Thr Trp Leu Gln Cys Ser Gly His Thr Asp Arg Cys
   420 425 430
   Asp Glu Ala Gln Val Leu Gln Val Trp Asp Asp Pro Tyr Pro Glu Val
   435 440 445
   Leu Ser Gln Glu Pro Phe His Lys Val Thr Val Gln Ser Leu Leu Thr
   450 455 460
   Val Glu Thr Leu Glu His Asn Gln Thr Tyr Glu Cys Arg Ala His Asn
   465 470 475 480
   Ser Val Gly Ser Gly Ser Trp Ala Phe Ile Pro Ile Ser Ala Gly Ala
   485 490 495
   His Thr His Pro Pro Asp Glu Phe Leu Phe Thr Pro Val Val Val Ala
   500 505 510
   Cys Met Ser Ile Met Ala Leu Leu Leu Leu Leu Leu Leu Leu Leu Leu
   515 520 525
   Tyr Lys Tyr Lys Gln Lys Pro Lys Tyr Gln Val Arg Trp Lys Ile Ile
   530 535 540
   Glu Ser Tyr Glu Gly Asn Ser Tyr Thr Phe Ile Asp Pro Thr Gln Leu
   545 550 555 560
   Pro Tyr Asn Glu Lys Trp Glu Phe Pro Arg Asn Asn Leu Gln Phe Gly
   565 570 575
   Lys Thr Leu Gly Ala Gly Ala Phe Gly Lys Val Val Glu Ala Thr Ala
   580 585 590
   Phe Gly Leu Gly Lys Glu Asp Ala Val Leu Lys Val Ala Val Lys Met
   595 600 605
   Leu Lys Ser Thr Ala His Ala Asp Glu Lys Glu Ala Leu Met Ser Glu
   610 615 620
   Leu Lys Ile Met Ser His Leu Gly Gln His Glu Asn Ile Val Asn Leu
   625 630 635 640
   Leu Gly Ala Cys Thr His Gly Gly Pro Val Leu Val Ile Thr Glu Tyr
   645 650 655
   Cys Cys Tyr Gly Asp Leu Leu Asn Phe Leu Arg Arg Lys Ala Glu Ala
   660 665 670
   Met Leu Gly Pro Ser Leu Ser Pro Gly Gln Asp Pro Glu Gly Gly Val
   675 680 685
   Asp Tyr Lys Asn Ile His Leu Glu Lys Lys Tyr Val Arg Arg Asp Ser
   690 695 700
   Gly Phe Ser Ser Gln Gly Val Asp Thr Tyr Val Glu Met Arg Pro Val
   705 710 715 720
   Ser Thr Ser Ser Asn Asp Ser Phe Ser Glu Gln Asp Leu Asp Lys Glu
   725 730 735
   Asp Gly Arg Pro Leu Glu Leu Arg Asp Leu Leu His Phe Ser Ser Gln
   740 745 750
   Val Ala Gln Gly Met Ala Phe Leu Ala Ser Lys Asn Cys Ile His Arg
   755 760 765
   Asp Val Ala Ala Arg Asn Val Leu Leu Thr Asn Gly His Val Ala Lys
   770 775 780
   Ile Gly Asp Phe Gly Leu Ala Arg Asp Ile Met Asn Asp Ser Asn Tyr
   785 790 795 800
   Ile Val Lys Gly Asn Ala Arg Leu Pro Val Lys Trp Met Ala Pro Glu
   805 810 815
   Ser Ile Phe Asp Cys Val Tyr Thr Val Gln Ser Asp Val Trp Ser Tyr
   820 825 830
   Gly Ile Leu Leu Trp Glu Ile Phe Ser Leu Pro Tyr Pro Gly Ile Leu
   835 840 845
   Val Asn Ser Lys Phe Tyr Lys Leu Val Lys Asp Gly Tyr Gln Met Ala
   850 855 860
   Gln Pro Ala Phe Ala Pro Lys Asn Ile Tyr Ser Ile Met Gln Ala Cys
   865 870 875 880
   Trp Ala Leu Glu Pro Pro Thr Phe Gln Gln Ile Cys Ser Phe Leu Gln
                   885                 890                 895
   Glu Gln Ala Gln Glu Asp Arg Arg Glu Arg Asp Tyr Thr Asn Leu Pro
               900                 905                 910
   Ser Ser Ser Arg Ser Gly Gly Ser Gly Ser Ser Ser Ser Glu Leu Glu
           915                 920                 925
   Glu Glu Ser Ser Ser Glu His Leu Thr Cys Cys Glu Gln Gly Asp Ile
       930                 935                 940
   Ala Gln Pro Leu Leu Gln Pro Asn Asn Tyr Gln Phe Cys
   945                 950                 955